(19) Europäisches Patentamt
European Patent Office
Office européen des brevets

(11) Veröffentlichungsnummer: 0 315 007
A1

(12) # EUROPÄISCHE PATENTANMELDUNG

(21) Anmeldenummer: 88117665.5

(22) Anmeldetag: 24.10.88

(51) Int. Cl.4: C07D 493/10 , C07D 405/06 , A01N 43/90 , //(C07D493/10, 311:00,307:00)

(30) Priorität: 03.11.87 DE 3737170

(43) Veröffentlichungstag der Anmeldung: 10.05.89 Patentblatt 89/19

(84) Benannte Vertragsstaaten: BE CH DE FR GB IT LI NL

(71) Anmelder: BAYER AG
Konzernverwaltung RP Patentabteilung
D-5090 Leverkusen 1 Bayerwerk(DE)

(72) Erfinder: Jautelat, Manfred, Dr.
Muellersbaum 28
D-5093 Burscheid(DE)
Erfinder: Elbe, Hans-Ludwig, Dr.
Dasnoeckel 59
D-5600 Wuppertal 11(DE)
Erfinder: Lürssen, Klaus, Dr.
August-Kierspel-Strasse 145
D-5060 Bergisch Gladbach 2(DE)

(54) **Azolyl-Spiroverbindungen.**

(57) Neue Azolyl-Spiroverbindungen der Formel

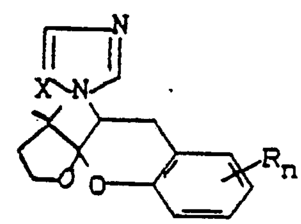

(I)

in welcher
R für Halogen, Alkyl, Alkoxy, Alkylthio, gegebenenfalls substituiertes Phenyl, gegebenenfalls substituiertes Phenoxy, Amino, Alkylamino und/oder Dialkylamino steht,
n für die Zahlen 0, 1, 2 oder 3 steht
und
X für Stickstoff oder eine CH-Gruppe steht,
sowie deren Säureadditions-Salze und Metallsalz-Komplexe, ein Verfahren zur Herstellung der neuen Wirkstoffe und deren Verwendung zur Regulierung des Pflanzenwachstums.

EP 0 315 007 A1

## Azolyl-Spiroverbindungen

Die vorliegende Erfindung betrifft neue Azolyl-Spiroverbindungen, ein Verfahren zu deren Herstellung und deren Verwendung als Pflanzenwachstumsregulatoren.

Es ist bereits bekannt, daß zahlreiche Azolyl-Derivate pflanzenwuchsregulierende Eigenschaften besitzen. Die Wirksamkeit dieser Stoffe ist im allgemeinen gut; bei niedrigen Aufwandmengen ist die pflanzenwuchsregulierende Aktivität allerdings nicht immer ausreichend.

Es wurden nun neue Azolyl-Spiroverbindungen der Formel

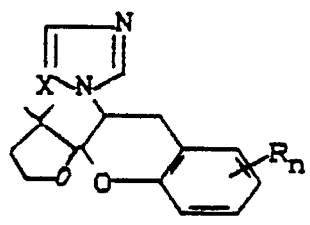

(I)

in welcher

R für Halogen, Alkyl, Alkoxy, Alkylthio, gegebenenfalls substituiertes Phenyl, gegebenenfalls substituiertes Phenoxy, Amino, Alkylamino und/oder Dialkylamino steht,

n für die Zahlen 0, 1, 2 oder 3 steht

und

X für Stickstoff oder eine CH-Gruppe steht,

sowie deren Säureadditions-Salze und Metallsalz-Komplexe gefunden.

Die erfindungsgemäßen Stoffe können je nach der Stellung des Azolyl-Restes in Bezug auf die Ebene des Tetrahydrofuran-Ringes in einer E-Form und einer Z-Form anfallen, wobei "E" für "entgegen" und "Z" für "zusammen" steht. Die Z-Form kann durch die Formel

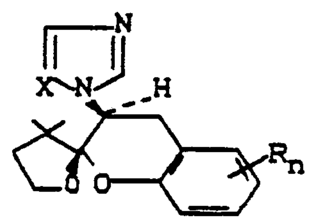

(I-Z)

wiedergegeben werden, während sich die E-Form durch die Formel

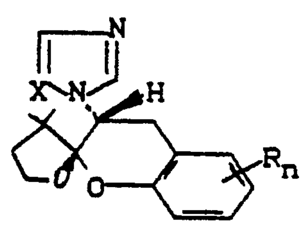

(I-E)

darstellen läßt. Die Erfindung betrifft sowohl die einzelnen E- und Z-Formen als auch deren Gemische.

Weiterhin wurde gefunden, daß man Azolyl-Spiroverbindungen der Formel (I) sowie deren Säureadditions-Salze und Metallsalz-Komplexe erhält, wenn man Azolylvinyl-Verbindungen der Formel

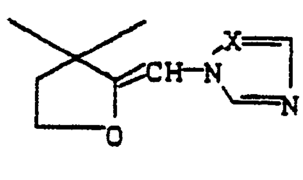

(II)

in welcher
X die oben angegebene Bedeutung hat,
mit Phenolen der Formel

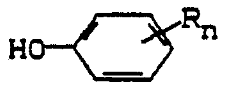

(III)

in welcher
R und n die oben angegebene Bedeutung haben,
in Gegenwart eines Katalysators sowie gegebenenfalls in Gegenwart eines Verdünnungsmittels umsetzt und die dabei entstehenden Tetrahydrofuran-Derivate der Formel

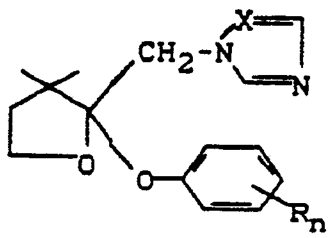

(IV)

in welcher
R, X und n die oben angegebene Bedeutung haben,
mit Formaldehyd in Gegenwart eines Katalysators sowie gegebenenfalls in Gegenwart eines Verdünnungsmittels umsetzt und gegebenenfalls anschließend an die so erhaltenen Verbindungen der Formel (I) eine Säure oder ein Metallsalz addiert.

Schließlich wurde gefunden, daß die neuen Azolyl-Spiroverbindungen der Formel (I) sowie deren Säureadditions-Salze und Metallsalz-Komplexe starke pflanzenwuchsregulierende Eigenschaften besitzen.

Überraschenderweise zeigen die erfindungsgemäßen Stoffe eine bessere pflanzenwuchsregulierende Wirksamkeit als die konstitutionell ähnlichsten vorbekannten Verbindungen gleicher Wirkungsrichtung.

Die erfindungsgemäßen Azolyl-Spiroverbindungen sind durch die Formel (I) allgemein definiert. In dieser Formel stehen vorzugsweise
R für Fluor, Chlor, Brom, Iod, Alkyl mit 1 bis 4 Kohlenstoffatomen, Alkoxy mit 1 bis 4 Kohlenstoffatomen, Alkylthio mit 1 bis 4 Kohlenstoffatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, Amino, Alkylamino mit 1 bis 4 Kohlenstoffatomen und/oder Dialkylamino mit 1 bis 4 Kohlenstoffatomen in jeder Alkylgruppe,
n für die Zahlen 0, 1, 2 oder 3 und
X für Stickstoff oder eine CH-Gruppe.

Wenn n für 2 oder 3 steht, können die für R stehenden Reste gleich oder verschieden sein.

Besonders bevorzugt sind diejenigen Verbindungen der Formel (I), in denen
R für Fluor, Chlor, Brom, Iod, Methyl, Ethyl, n-Propyl, iso-Propyl, n-Butyl, sek.-Butyl, iso-Butyl, tert.-Butyl, Methoxy, Ethoxy, Methylthio, Ethylthio, gegebenenfalls einfach oder zweifach, gleichartig oder verschieden durch Fluor, Chlor, Brom und/oder Methyl substituiertes Phenyl, gegebenenfalls einfach oder zweifach, gleichartig oder verschieden durch Fluor, Chlor, Brom und/oder Methyl substituiertes Phenoxy, Amino, Methylamino, Ethylamino, Dimethylamino und/oder Diethylamino steht,
n für die Zahlen 0, 1 oder 2 steht und
X für Stickstoff oder eine CH-Gruppe steht.

Bevorzugte erfindungsgemäße Verbindungen sind auch Additionsprodukte aus Säuren und denjenigen Azolyl-Spiroverbindungen der Formel (I), in denen R, X und n die Bedeutungen haben, die bereits vorzugsweise für die Reste bzw. diesen Index genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Azolyl-Spiroverbindungen der Formel (I), in denen R, X und n die Bedeutungen haben, die bereits vorzugsweise für diese Reste bzw. diesen Index genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen.

Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für Azolyl-Spiroverbindungen der Formel (I) seien die in der folgenden Tabelle aufgeführten Stoffe genannt.

<u>Tabelle 1</u>

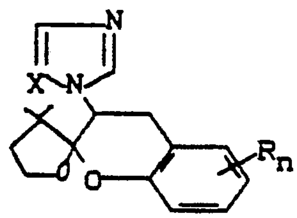

(I)

| $R_n$ | X | $R_n$ | X |
|---|---|---|---|
| 4-Cl | CH | 4-O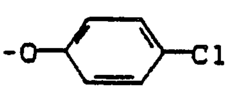 | CH |
| 4-Cl, 6-$CH_3$ | CH | 4-$NH_2$ | N |
| 4-F | CH | 4-$NH_2$ | CH |
| 4,6-$Cl_2$ | CH | 4-$NHCH_3$ | N |
| 4-$OCH_3$ | CH | 4-$NHCH_3$ | CH |
| 4-$SCH_3$ | N | 4-$N(CH_3)_2$ | N |
| 4-$CH_3$ | N | 4-$N(CH_3)_2$ | CH |
| 4,6-$(CH_3)_2$ | N | | |
| 4-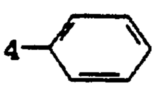 | N | | |
| 4-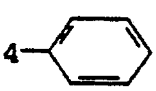 | CH | | |
| 4-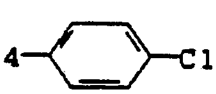 | N | | |
| 4-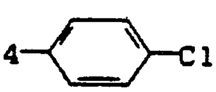 | CH | | |

Tabelle 1 (Fortsetzung)

| $R_n$ | X |
|---|---|
| 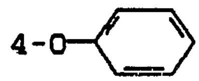 | N |
| 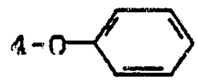 | CH |
| 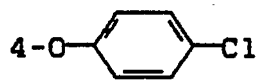 | N |

Verwendet man (3.3-Dimethyl-tetrahydrofuran-2-yliden)-(1,2,4-triazol-1-yl)-methan und 4-Chorphenol als Ausgangsstoffe und Paraformaldehyd als Reaktionskomponente, so kann der Verlauf des erfindungsgemäßen Verfahrens durch das folgende Formelschema veranschaulicht werden:

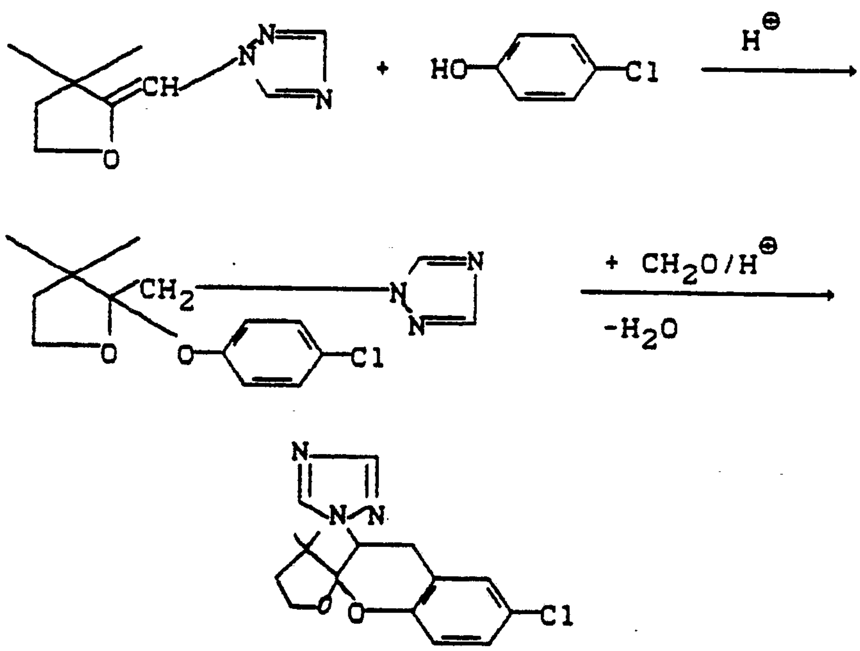


Die bei dem erfindungsgemäßen Verfahren als Ausgangsstoffe benötigten Azolyl-vinyl-Verbindungen sind durch die Formel (II) definiert. In dieser Formel steht X für Stickstoff oder eine CH-Gruppe.

Die Azolyl-vinyl-Verbindungen der Formel (II) sind bereits bekannt (vgl. EP-OS 0 203 504).

Die bei dem erfindungsgemäßen Verfahren weiterhin als Ausgangsprodukte benötigten Phenole sind durch die Formel (III) allgemein definiert. In dieser Formel haben R und n vorzugsweise diejenigen Bedeutungen, die bereits im Zusammenhang mit der Beschreibung der erfindungsgemäßen Stoffe der Formel (I) vorzugsweise für diesen Rest bzw. diesen Index genannt wurden.

Die Phenole der Formel (III) sind bekannt oder lassen sich nach prinzipiell bekannten Verfahren herstellen.

Die bei der Durchführung des erfindungsgemäßen Verfahrens als Zwischenprodukte anfallenden Tetrahydrofuran-Derivate der Formel (IV) sind neu.

Als Reaktionskomponente bei der Durchführung des erfindungsgemäßen Verfahrens dient Formaldehyd. Dieser kann in trimerer Form als Trioxan oder auch in polymerer Form als Paraformaldehyd eingesetzt werden.

Als Katalysatoren können bei der Durchführung des erfindungsgemäßen Verfahrens sowohl in der

ersten als auch in der zweiten Stufe alle für derartige Umsetzungen üblichen sauren Reaktionsbeschleuniger eingesetzt werden. Vorzugsweise verwendbar sind starke anorganische und organische Säuren, wie Chlorwasserstoffsäure, Trifluoressigsäure und Toluolsulfonsäure.

Bei der Durchführung des erfindungsgemäßen Verfahrens arbeitet man sowohl in der ersten Stufe als auch in der zweiten Stufe entweder in Abwesenheit von zusätzlichen Verdünnungsmitteln oder in Anwesenheit von inerten Verdünnungsmitteln. Vorzugsweise in Frage kommen dabei inerte organische Solventien, beispielsweise Ether, wie Tetrahydrofuran und Dioxan, ferner Ester, wie Essigsäurebutylester, weiterhin Säuren, wie Essigsäure oder Propionsäure und außerdem Nitrile, wie Acetonitril und Propionitril.

Die Reaktionstemperaturen können bei dem erfindungsgemäßen Verfahren sowohl bei der Durchführung der ersten als auch der zweiten Stufe innerhalb eines größeren Bereiches variiert werden. Im allgemeinen arbeitet man jeweils bei Temperaturen zwischen 50 °C und 150 °C, vorzugsweise zwischen 60 °C und 120 °C.

Das erfindungsgemäße Verfahren wird im allgemeinen unter Normaldruck durchgeführt. Es ist jedoch auch möglich unter erhöhtem oder vermindertem Druck zu arbeiten.

Bei der Durchführung des erfindungsgemäßen Verfahrens setzt man auf 1 Mol an Azolylvinyl-Verbindungen der Formel (II) im allgemeinen 1 bis 2 Mol, vorzugsweise 1 bis 1,5 Mol an Phenol der Formel (III) sowie 1 bis 4 Mol, vorzugsweise 1,5 bis 2,5 Mol, an Formaldehyd und außerdem 0,1 bis 1,5 Mol an Katalysator ein. Die Aufarbeitung erfolgt nach üblichen Methoden.

Das erfindungsgemäße Verfahren kann in der Weise durchgeführt werden, daß man zunächst das Tetrahydrofuran-Derivat der Formel (IV) herstellt und dieses dann in einem gesonderten Schritt zum Endprodukt umsetzt. Es ist jedoch auch möglich, nach einem Eintopf-Verfahren zu arbeiten.

Die erfindungsgemäßen Azolyl-Spiroverbindungen fallen bei der Herstellung im allgemeinen in Formel von E/Z-Isomerengemischen an. Diese Gemische lassen sich nach üblichen Methoden, z.B. auf chromatographischem Wege, in die einzelnen Komponenten zerlegen.

Die E- und Z-Formen der Azolyl-Spiroverbindungen der Formel (I) unterscheiden sich in ihren $^1$H-Kernresonanz-Spektren in charakteristischer Weise. Im Falle der Z-Formen liegt die Kopplungskonstante des H-Atoms an dem Kohlenstoffatom, das den Azolyl-Rest trägt, zu den beiden Wasserstoffatomen der benachbarten $CH_2$-Gruppe im Sechsring bei etwa 7 bis 9 Hz, während das entsprechende Wasserstoffatom im Falle der E-Formen eine Kopplungskonstante von etwa 6 Hz aufweist.

Die nach dem erfindungsgemäßen Verfahren erhältlichen Azolyl-Spiroverbindungen der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt werden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die

Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten-und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeit punkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin könne Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine

Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die erfindungsgemäßen Wirkstoffe eignen sich vorzugsweise zur Hemmung des Pflanzenwachstums. Mit besonderem Vorteil lassen sie sich zur Hemmung des Wachstums von Dikotylen, wie Raps, Sojabohnen und Baumwolle, verwenden.

Die erfindungsgemäßen Wirkstoffe besitzen auch eine fungizide Wirksamkeit. Sie lassen sich besonders vorteilhaft gegen Pilzbefall in Getreide sowie zur Bekämpfung von Botrytis einsetzen.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtiono gene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten

Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe gehen aus den nachfolgenden Beispielen hervor.

Herstellungsbeispiele

Beispiel 1

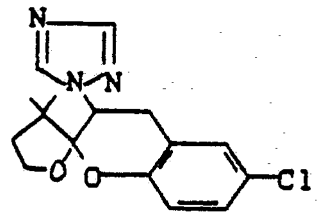

(I-1)
E/Z-Gemisch

Ein Gemisch aus 9,0 g (0,05 Mol) (3,3-Dimethyltetrahydrofuran-2-yliden)-(1,2,4-triazol-1-yl)-methan, 6,4 g (0,05 Mol) 4-Chlorphenol, 3,0 g (0,1 Mol) Paraformaldehyd und 1 ml Trifluoressigsäure wird 22 Stunden auf 100 °C erhitzt. Man fügt dann noch 1,5 g (0,05 Mol) Paraformaldehyd hinzu und erhitzt weitere 20 Stunden auf 100 °C. Nach dem Abkühlen auf Raumtemperatur wird das Reaktionsgemisch mit Methylenchlorid verdünnt und mehrfach mit 2N wäßriger Natronlauge ausgeschüttelt. Die organische Phase wird abgetrennt und nach dem Trocknen durch Abziehen des Lösungsmittels unter vermindertem Druck eingeengt. Man erhält auf diese Weise 13,2 g (82 % der Theorie) an der oben bezeichneten Spiro-Verbindung in Form eines Gemisches E:Z = 1,9:1. Schmelzbereich: 125-150 °C.

Herstellung des Ausgangsproduktes

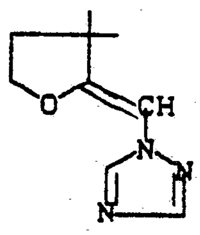

85 g (1,23 Mol) 1,2,4-Triazol und 464 g (33,6 Mol) Kaliumcarbonat werden in 2,5 l Aceton vorgelegt. Bei Raumtemperatur läßt man ohne Kühlung unter Rühren 220 g (1,12 Mol) 1,5-Dichlor-3,3-dimethyl-2-pentanon zutropfen. Nach Beendigung der Zugabe läßt man 2 Stunden bei Rückflußtemperatur nachrühren.

Das Reaktionsgemisch wird abgekühlt, über eine Nutsche abgesaugt, und die Mutterlauge wird unter vermindertem Druck eingeengt. Der Rückstand wird in Methylenchlorid aufgenommen. Die Lösung wird mit Wasser gewaschen, über Natriumsulfat getrocknet und eingeengt. Der verbleibende Rückstand wird in Petrolether verrührt, abgesaugt und im Vakuum bei 50 °C getrocknet.

Man erhält 145,3 g (56,5 % der Theorie) (3,3-Dimethyltetrahydrofuran-2-yliden)-(1,2,4-triazol-1-yl)-methan vom Schmelzpunkt 47 °C.

Beispiele 2 und 3

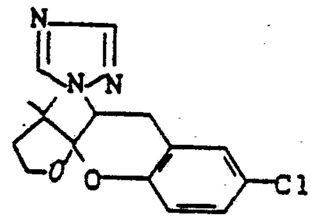

(I-2)

(E-Form)

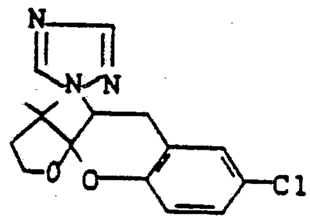

(I-3)

(Z-Form)

Das E/Z-Gemisch (I-1) wird durch Chromatographie über Kieselgel mit Essigsäureethylester als Eluierungsmittel in die einzelnen Isomeren zerlegt. Man erhält dabei die folgenden Komponenten:

1. E-Isomeres der Formel (I-2)

Schmelzpunkt: 132-134 °C

$^1$H-NMR ($CDCl_3$)δ: 0,56 (s, 3 H), 1,25 (s, 3 H), 1,75 (m, 1 H), 2,35 (m, 1 H), 2,84 (d, J = 17,5 Hz, 1 H), 3,65 (2 d, J = 17,5 u. 6 Hz, 1 H), 3,95 (m, 2 H), 5,14 (d, J = 6 Hz, 1 H), 6,92 (d, J = 8,5 Hz, 1 H), 7,12 (d, J = 2 Hz, 1 H), 7,22 (2d, J = 2 u. 8,5 Hz, 1 H), 8,0 (s, 1 H), 8,05 (s, 1 H).

2. Z-Isomeres der Formel (I-3)

Schmelzpunkt: 170-173 °C

$^1$H-NMR ($CDCl_3$)δ: 0,8 (s, 1 H), 1,15 (s, 1 H), 1,75 (m, 1 H), 2,4 (m, 1 H), 3,3 (d, J = 7 Hz, 1 H), 3,35 (d, J = 9 Hz, 1 H), 4,1 (m, 2 H); 5,0 (2 d, J = 7 u. 9 Hz, 1 H), 6,8 (d, J = 8,5, 1 H), 7,2 (d, J = 3,5 Hz, 1 H); 7,15 ( 2d, J = 3,5 u. 8,5 Hz, 1 H), 8,1 (s, 1 H), 8,7 (s, 1 H).

Beispiel 4

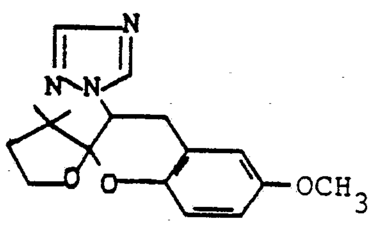

(I-4)

E/Z-Gemisch

Ein Gemisch aus 3,6 g (20 mMol) (3,3-Dimethyl-tetrahydrofuran-2-yliden)-(1,2,4-triazol-1-yl)-methan, 2,5 g (20 mMol) 4-Methoxyphenol, 0,6 g (20 mMol) Paraformaldehyd, 1 ml Trifluoressigsäure und 50 ml Dioxan wird 192 Stunden unter Rückfluß erhitzt, wobei weitere 1,2 g (40 mMol) Paraformaldehyd in Portionen von 0,2 g alle 24 Stunden hinzugefügt werden. Nach dem Abkühlen auf Raumtemperatur wird das Reaktionsgemisch mit Methylenchlorid verdünnt und mehrfach mit 2N wäßriger Natronlauge ausgeschüttelt. Die organische Phase wird abge trennt und nach dem Trocknen durch Abziehen des Lösungsmittels unter vermindertem Druck eingeengt. Man erhält 5,4 g eines Produktes, das zur weiteren Reinigung über 50 g Kieselgel mit Ethylacetat filtriert wird. Nach dem Abziehen des Lösungsmittels verbleiben 4,5 g (72,5 % der Theorie) an der oben bezeichneten Spiro-Verbindung in Form eines Gemisches E:Z = 0,5:1 mit einem Schmelzpunkt von 127-130 °C.

Nach den in den Beispielen 1 und 3 angegebenen Methoden werden auch die in der folgenden Tabelle 2 formelmäßig aufgeführten Stoffe hergestellt.

Tabelle 2

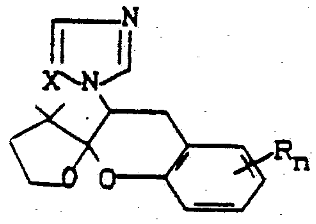

(I)

| Beispiel Nr. | Verbindung | $R_n$ | X | E:Z-Verhältnis |
|---|---|---|---|---|
| 5 | I-5 | 4-Cl, 6-$CH_3$ | N | 0,32:1 |
| 6 | I-6 | 4-F | N | 2,6 :1 |
| 7 | I-7 | 4,6-$Cl_2$ | N | 0,22:1 |

Die in den Beispielen 5-7 aufgeführten E/Z-Gemische fallen in Form von öligen Substanzen an, die durch ihre $^1$H-NMR-Spektren eindeutig charakterisiert wurden.

Beispiel A

Wachstum bei Baumwolle

| | |
|---|---|
| Lösungsmittel: | 30 Gewichtsteile Dimethylformamid |
| Emulgator: | 1 Gewichtsteil Polyoxyethylen-Sorbitan-Monolaurat |

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 2 Wochen wird der Zuwachs bei allen Pflanzen gemessen und das Wachstum in Prozent des Wachstums der Kontrollpflanzen berechnet. Es bedeuten 100 % Wachstum ein Wachstum entsprechend dem der Kontrollpflanzen und 0 % den Stillstand des Wachstums. Werte über 100 % kennzeichnen eine Wuchsförderung.

In diesem Test zeigen die in den Beispielen (1) und (5) beschriebenen erfindungsgemäßen Wirkstoffe eine sehr starke Wuchshemmung.

Beispiel B

Wachstum bei Sojabohnen

| | |
|---|---|
| Lösungsmittel: | 30 Gewichtsteile Dimethylformamid |
| Emulgator: | 1 Gewichtsteil Polyoxyethylen-Sorbitan-Monolaurat |

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 2 Wochen wird bei allen Pflanzen der Zuwachs gemessen und das Wachstum in Prozent des Wachstums der Kontrollpflanzen berechnet. Es bedeuten 100 % Wachstum ein Wachstum entsprechend dem der Kontrollpflanzen und 0 % den Stillstand des Wachstums. Werte über 100 % kennzeichnen eine Wuchsförderung.

In diesem Test zeigen die in den Beispielen (1), (5) und 6 beschriebenen erfindungsgemäßen Stoffe eine sehr starke wuchshemmende Wirkung.

Beispiel C

Wachstum bei Raps

| | |
|---|---|
| Lösungsmittel: | 30 Gewichtsteile Dimethylformamid |
| Emulgator: | 1 Gewichtsteil Polyoxyethylen-Sorbitan-Monolaurat |

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Rapspflanzen werden im Gewächshaus bis zum Erscheinen des 5. Blattes angezogen. In diesem Stadium werden die Pflanzen tropfnaß mit den Wirkstoffzubereitungen besprüht. Nach 2 Wochen wird der Zuwachs der Pflanzen gemessen und das Wachstum in Prozent des Wachstums der Kontrollpflanzen berechnet. Es bedeuten 100 % Wachstum ein Wachstum entsprechend dem der Kontrollpflanzen und 0 % den Stillstand des Wachstums. Werte über 100 % kennzeichnen eine Wuchsförderung.

In diesem Test zeigt der im Beispiel (1) beschriebene erfindungsgemäße Wirkstoff eine sehr starke wuchhemmende Wirkung.

Beispiel D

Wachstum bei Mais

| | |
|---|---|
| Lösungsmittel: | 30 Gewichtsteile Dimethylformamid |
| Emulgator: | 1 Gewichtsteil Polyoxyethylen-Sorbitan-Monolaurat |

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Maispflanzen werden im Gewächshaus bis zum Zweiblatt-Stadium angezogen. In diesem Stadium werden die Pflanzen tropfnaß mit den Wirkstoffzubereitungen besprüht. Nach zwei Wochen wird bei allen Pflanzen der Zuwachs gemessen und das Wachstum in Prozent des Zuwachses der Kontrollpflanzen berechnet. Es bedeuten 100 % Wachstum ein Wachstum entsprechend dem der Kontrollpflanzen und 0 % den Stillstand des Wachstums. Werte über 100 % kennzeichnend eine Wuchsförderung.

In diesem Test zeigt der im Beispiel (1) beschriebene erfindungsgemäße Wirkstoff eine sehr starke wuchshemmende Wirkung.

## Ansprüche

1. Azolyl-Spiroverbindungen der Formel

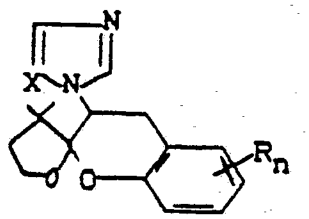

(I)

in welcher
R für Halogen, Alkyl, Alkoxy, Alkylthio, gegebenenfalls substituiertes Phenyl, gegebenenfalls substituiertes Phenoxy, Amino, Alkylamino und/oder Dialkylamino steht,
n für die Zahlen 0, 1, 2 oder 3 steht
und
X für Stickstoff oder eine CH-Gruppe steht,
sowie deren Säureadditions-Salze und Metallsalz-Komplexe.

2. Azolyl-Spiroverbindungen der Formel (I) gemäß Anspurch 1, in denen
R für fluor, Chlor, Brom, Iod, Alkyl mit 1 bis 4 Kohlenstoffatomen, Alkoxy mit 1 bis 4 Kohlenstoffatomen, Alkylthio mit 1 bis 4 Kohlenstoffatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, Amino, Alkylamino mit 1 bis 4 Kohlenstoffatomen und/oder Dialkylamino mit 1 bis 4 Kohlenstoffatomen in jeder Alkylgruppe steht,
n für die Zahlen 0, 1, 2 oder 3 steht und
X für Stickstoff oder eine CH-Gruppe steht.

3. Verfahren zur Herstellung von Azolyl-Spiroverbindungen der Formel

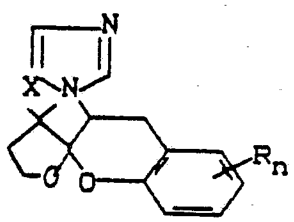

(I)

in welcher
R für Halogen, Alkyl, Alkoxy, Alkylthio, gegebenenfalls substituiertes Phenyl, gegebenenfalls substituiertes Phenoxy, Amino, Alkylamino und/oder Dialkylamino steht,
n für die Zahlen 0, 1, 2 oder 3 steht
und
X für Stickstoff oder eine CH-Gruppe steht,
sowie von deren Säureadditions-Salzen und Metallsalz-Komplexen, dadurch gekennzeichnet, daß man Azolyl-vinyl-Verbindungen der Formel

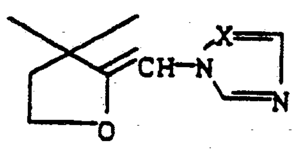

(II)

in welcher
X die oben angegebene Bedeutung hat,
mit Phenolen der Formel

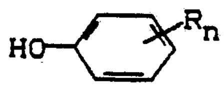

(III)

in welcher
R und n die oben angegebene Bedeutung haben,
in Gegenwart eines Katalysators sowie gegebenenfalls in Gegenwart eines Verdünnungsmittels umsetzt und die dabei entstehenden Tetrahydrofuran-Derivate der Formel

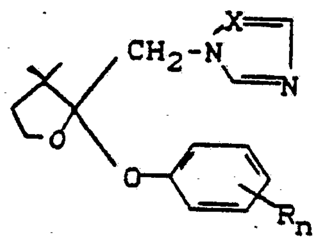

(IV)

in welcher
R, X und n die oben angegebene Bedeutung haben,
mit Formaldehyd in Gegenwart eines Katalysators sowie gegebenenfalls in Gegenwart eines verdünnungsmittels umsetzt und gegebenenfalls anschließend an die so erhaltenen Verbindungen der Formel (I) eine Säure oder ein Metallsalz addiert.

4. Pflanzenwuchsregulierende Mittel, gekennzeichnet durch einen Gehalt an mindestens einer Azolyl-Spiroverbindung der Formel (I) gemäß Anspruch 1 bzw. an einem Säureadditionssalz oder Metallsalz-Komplex einer Azolyl-Spiroverbindung der Formel (I).

5. Verwendung von Azolyl-Spiroverbindungen der Formel (I) gemäß Anspruch 1 bzw. von deren Säureaddi tionssalzen oder Metallsalz-Komplexen zur Regulierung des Pflanzenwachstums.

6. Verfahren zur Regulierung des Pflanzenwachstums, dadurch gekennzeichnet, daß man Azolyl-Spiroverbindungen der Formel (I) gemäß Anspruch 1 bzw. deren Säureadditionssalze oder Metallsalz-Komplexe auf die Pflanzen und/oder deren Lebensraum ausbringt.

7. Verfahren zur Herstellung von pflanzenwuchsregulierenden Mitteln, dadurch gekennzeichnet, daß man Azolyl-Spiroverbindungen der Formel (I) gemäß Anspruch 1 bzw. deren Säureadditionssalze oder Metallsalz-Komplexe mit Streckmitteln und/oder oberflächenaktiven Stoffen vermischt.

8. Tetrahydrofuran-Derivate der Formel

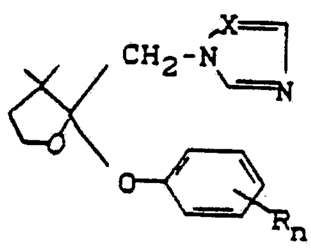

(IV)

in welcher
R für Halogen, Alkyl, Alkoxy, Alkylthio, gegegebenenfalls substituiertes Phenyl, gegebenenfalls substituiertes Phenoxy, Amino, Alkylamino und/oder Dialkylamino steht,
n für die Zahlen 0, 1, 2 oder 3 steht
und
X für Stickstoff oder eine CH-Gruppe steht.

Europäisches Patentamt

# EUROPÄISCHER RECHERCHENBERICHT

Nummer der Anmeldung

EP 88 11 7665

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 023 756 (ICI)<br>* Ansprüche *<br>--- | 1,2,4-7 | C 07 D 493/10<br>C 07 D 405/06<br>A 01 N 43/90 //<br>(C 07 D 493/10<br>C 07 D 311:00<br>C 07 D 307:00 ) |
| A | EP-A-0 142 684 (BAYER)<br>* Ansprüche; Seiten 32,37 *<br>----- | 8 | |
| | | | **RECHERCHIERTE SACHGEBIETE (Int. Cl.4)** |
| | | | C 07 D 405/00<br>C 07 D 493/00 |

Der vorliegende Recherchenbericht wurde für alle Patentansprüche erstellt

| Recherchenort | Abschlußdatum der Recherche | Prüfer |
|---|---|---|
| DEN HAAG | 30-01-1989 | WRIGHT M.W. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument